# EUROPEAN PATENT APPLICATION

(11) **EP 4 129 616 A1**
(43) Date of publication of application: **08.02.2023**
(21) Application number: 21780697.5
(22) Date of filing: 31.03.2021
(51) Int. Cl.: B29C 45/00, B29C 45/27, B29C 45/37, A61M 5/28, A61M 5/31

(54) **MEDICAL SYRINGE BARREL AND METHOD FOR PRODUCING SAME**

(30) Priority: 31.03.2020 JP 2020062640
(71) Applicant: Daikyo Seiko, LTD., Sano-shi, Tochigi 327-0813 (JP)
(72) Inventor: YOSHIDA Takayuki, Sano-shi, Tochigi 327-0813 (JP)
(74) Representative: Grünecker Patent- und Rechtsanwälte PartG mbB
(86) International application number: PCT/JP2021/013774
(87) International publication number: WO 2021/201073

(57) **Abstract**

**Problem**

To provide a medical syringe barrel and a manufacturing method for producing the same which can be completed as a molded article by injection molding of a synthetic resin material without requiring post processing like polishing and can fill and administer a predetermined amount of the internal-use solution accurately.

**Solution**

A medical syringe barrel comprising a tubular barrel section for accommodating an internal-use solution in the interior thereof, an injection nozzle section formed at one end of the barrel section, and a flange section formed at an opening section on the other end of the barrel section to project radially outward, wherein the surface of the flange section has a recess portion, and a weld line which is potentially formed by injection molding is not formed in the internal-use solution filled section.

## Description

### Technical Field

The present invention relates to a medical syringe barrel for constituting a medical syringe and a manufacturing method for producing the same.

### Background Art

In general, a medical syringe comprises a syringe barrel having a tip on which a needle is to be attached and a syringe plunger which is to be inserted into the syringe barrel from an opening on the other end for moving a piston in the axial direction. A flange is formed at the opening on the other end of the syringe barrel to project radially outward.

For simplifying medicine management, controlling infection, preventing errors at the time of dispensing, speeding up administration and so on, a prefilled syringe, in which an accurate dosage of medicine is previously filled, is used. Plastic syringes are widely used because they have advantages of being highly flexible in the shape, lightweight, capable of preventing cracks, easy to dispose by fire and so on, and they improve the usability at pharmaceutical companies and medical institutions.

In general, such plastic syringes are manufactured by injection molding of a synthetic resin material. The injection molding is a typical plastic molding method which can produce products having complicated and precise shapes in a short time and at a low cost by mass production. This is a processing method for obtaining a molded article having a desired shape by heating and melting a synthetic resin material, applying pressure to the material, injecting it into a closed mold, cooling it, opening the mold and taking the solidified article out from the mold.

Patent Document 1 discloses a technique of installing a gate for injecting a synthetic resin material at the shaft center to form a rotating body shaped molded article by a synthetic resin material using a mold. Patent Document 2 proposes a technique of preventing a weld line from being formed in the blood contacting area of a blood equipment cap.

Patent Document 1: Japanese laid-open patent application 2001-121546
Patent Document 2: Japanese laid-open patent application 2019-150588

### Disclosure of Invention

### Problems to be resolved by the Invention

Conventionally, as shown in Patent Document 1, when a syringe barrel is manufactured by injection molding of a synthetic resin material, a gate is installed at the shaft center, namely an injection nozzle section of a syringe barrel, because the mold can be easily manufactured. In a molded article by injection molding of a synthetic resin material, a small groove or recess known as a weld line, which extends linearly, is formed at a position where the streams of the melted resin material join together inside the mold. Because a syringe barrel is a hollow structured molded article with a hole bored in the center, an injected melted resin material divides into two streams at a position, where a hollow structured hole is formed, and the streams join together thereafter. In such a syringe barrel, a weld line is formed at a position which is close to the injection nozzle section between the injection nozzle section and the barrel section.

If a weld line is formed at an internal liquid filled section in which an internal-use solution is filled, a groove shaped gap is formed between the outer circumferential surface of a piston and the inner wall surface of a barrel section, and there is some possibility of running of the internal-use solution out from the internal-use solution filled section along the gap. There is also a possibility that an internal-use solution remains in the weld line at the time of using the syringe. When a minimal capacity syringe is used, it is necessary to administer a regulated amount of the internal-use solution accurately, and therefore it is necessary to prevent such a leakage of the internal-use solution or a residual internal-use solution. When the side wall of the internal-use solution filled section of a barrel is tapped by a medical worker for discharging air bubbles at the time of using a syringe, the weld line can be a cause of a breakage like a crack on the barrel.

It is also necessary for a medical syringe barrel for a prefilled syringe to receive a sterilization treatment, be packaged and then be transported to a pharmaceutical company without being subjected to post processing like polishing after injection molding in order to prevent contamination.

### Means for solving the Problems

(1) A medical syringe barrel according to the present invention comprises a tubular barrel section for accommodating an internal-use solution in the interior thereof, an injection nozzle section formed at one end of the barrel section, and a flange section which is formed at an opening section on the other end of the barrel section to project radially outward,
The barrel section includes an internal-use solution filled section which is set to be filled with the internal-use solution and an internal-use solution unfilled section which is set not to be filled with the internal-use solution when the internal-use solution is poured into the syringe barrel from the opening section. The surface of the flange section has a recess portion, and a weld line which is potentially formed by injection molding is not formed in the internal-use solution filled section.

According to a medical syringe barrel which is configured as described above, a weld line by injection molding of a synthetic resin material is formed at a position which is close to the flange section between the flange section and the barrel section, where melted resin streams join together on the circumference. However, no weld line is formed at the internal-use solution filled section, namely the portion which is close to the injection nozzle section in the barrel section. Therefore, it is possible to prevent formation of a gap by the weld line between the outer circumferential surface of the piston and the inner wall surface of the syringe barrel, and therefore it is possible to provide a syringe barrel in which a predetermined amount of the internal-use solution can be filled and administered accurately, when the internal-use solution is filled in the syringe barrel.

(2) In a medical syringe barrel according to the present invention described in (1), the height of a gate rest which is potentially formed by injection molding is smaller than the depth of the recess portion of the flange section, and the gate rest fits inside the recess portion.

It becomes unnecessary to conduct post processing like polishing by configuring the recess portion which is formed in the flange section as described above, because the gate rest fits inside the recess portion certainly, although the projection by the gate rest may cause an injury at the time of use by a medical worker and it may cause improper mounting of a finger grip. Therefore, it is possible to provide a medical syringe barrel which is suitable for a hygienic full-automatic manufacturing and packaging process which can prevent contamination by foreign substances.

(3) A manufacturing method for producing a medical syringe barrel according to the present invention is a method for manufacturing a medical syringe barrel which comprises a tubular barrel section for accommodating an internal-use solution in the interior thereof, an injection nozzle section formed at one end of the barrel section, and a flange section which is formed at an opening section on the other end of the barrel section to project radially outwa rd,
In the present invention, injection molding of a thermoplastic resin material is conducted by preparing a mold so that a recess portion is formed on the surface of the flange section and installing a gate at a position where the recess portion is to be formed.

By configuring a medical syringe barrel in this way, a weld line by injection molding of a synthetic resin material is potentially formed at a position which is close to the flange section between the flange section and the barrel position, where the streams of a melted resin material join together on the circumference. However, no weld line is formed in the internal-use solution filled section, namely at the position which is close to the injection nozzle section in the barrel section. Therefore, it is possible to manufacture a syringe barrel which can prevent a gap by a weld line from forming between the outer circumference surface of the piston and the inner wall surface of the syringe barrel, and therefore can fill and administer a predetermined amount of the internal-use solution accurately, when the internal-use solution is filled in the syringe barrel. It is also possible to complete a syringe barrel without requiring any post processing like polishing for removing gate rests because the surface of the flange section is formed so as to have a recess portion.

### Effect of Invention

In a medical syringe barrel and a manufacturing method for producing the same according to the present invention, it is possible to provide a syringe barrel which can be completed as a molded article by injection molding of a synthetic resin material without requiring post processing like polishing and can fill and administer a predetermined amount of the internal-use solution accurately.

### Brief Description of Drawings

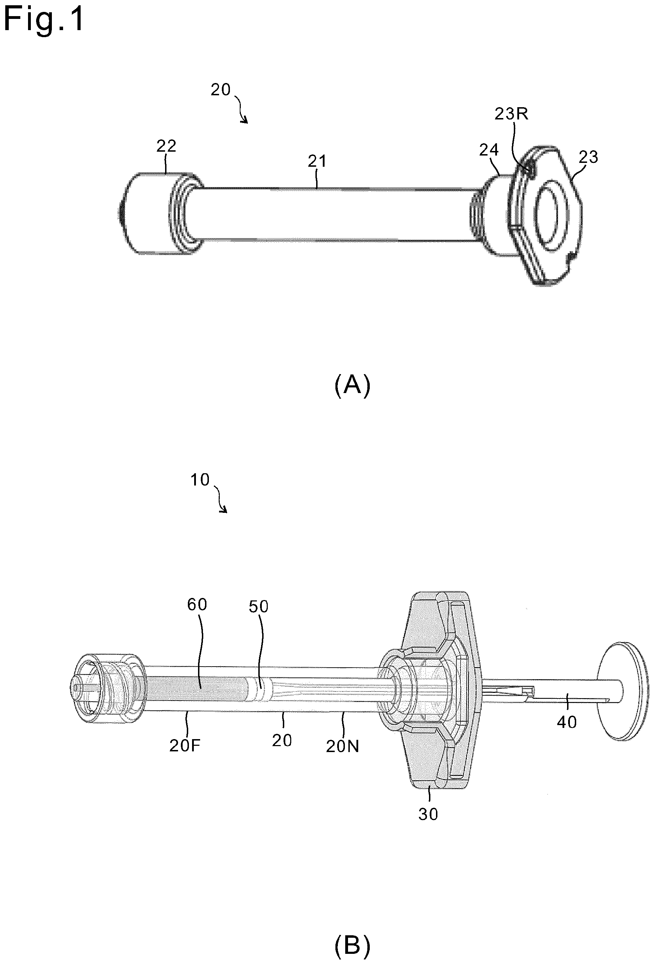

Fig. 1 (A) shows a perspective view of a schematic configuration of a syringe barrel according to one embodiment of the present invention, and (B) shows a perspective view of a configuration example of a medical syringe made by assembling a syringe barrel shown in (A), in which an internal-use solution is filled, together with a piston, a plunger and a finger grip.

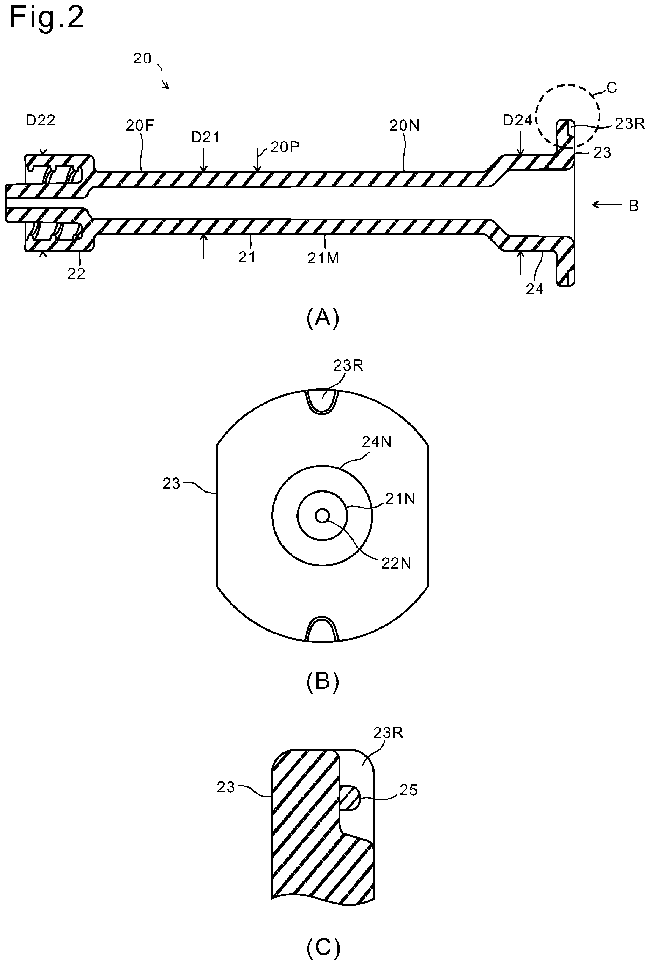

Fig.2 (A) shows an axial cross-sectional view of a syringe barrel according to one embodiment of the present invention, (B) shows a side view observed from the arrow B in (A), and (C) shows an enlarged view of the portion C surrounded by the dotted line in (A).

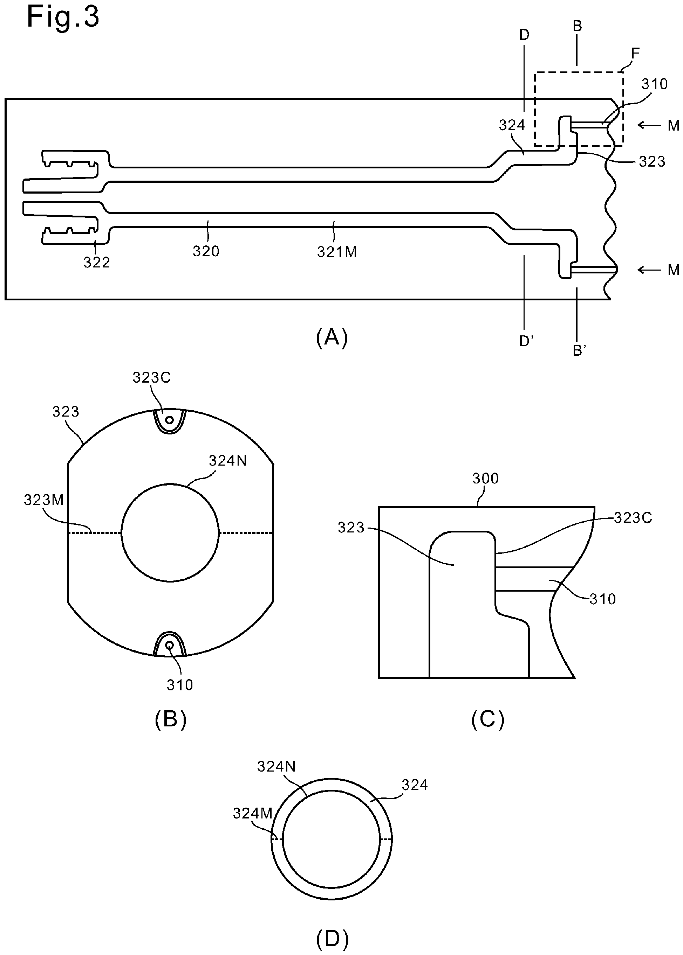

Fig. 3 shows a schematic cross-sectional view of a mold and a gate for explaining a manufacturing method for producing the syringe barrel shown in Fig.2, (A) shows an axial cross-sectional view of the mold, (B) shows a B-B' cross sectional view of the mold shown in (A), (C) shows an enlarged cross-sectional view of the portion F of the mold in (A), and (D) shows a D-D' cross sectional view of the mold shown in (A).

### A Mode for implementing the Invention

A configuration of a medical syringe barrel according to one embodiment of the present invention will be described below referring to the drawings. In Fig. 1(A), a syringe barrel 20 is a molded article by injection molding of a synthetic resin material like a polyolefin resin material, and comprises a tubular barrel section 21 for accommodating an internal-use solution in the interior thereof, a Lure Lock type injection nozzle section 22 which is formed at one end of the barrel section 21 and to which a needle as an example is to be attached, and a flange section 23 which is formed at an opening section on the other end of the barrel section 21 to project radially outward. The syringe barrel 20 is a transparent molded article, however only the outer shape is shown in Fig.1(A).

Fig.1 (B) shows a perspective view of a configuration example of a medical syringe which is assembled by a syringe barrel 20 according to one embodiment of the present invention in which an internal-use solution is filled, together with a piston, a plunger and a finger grip. This medical syringe is a prefilled syringe with a minimal capacity for ophthalmic use as an example, and a disposable syringe for which a single time use is assumed. In Fig.1(B), a syringe 10 is an assembly which is made of a syringe barrel 20, a finger grip 30, a syringe plunger 40 and a piston 50. An internal-use solution 60 is accommodated in a hollow barrel section of the tubular syringe barrel 20, the internal-use solution 60 is pushed out from the tip of the syringe barrel 20 by pushing the piston 50 with the tip of the syringe plunger 40. As shown in Fig. 1(B), the syringe barrel 20 is a transparent barrel of a molded article made of a polyolefin resin material as an example, and it is possible to visually recognize the internal-use solution 60, the piston 50 and a part of the syringe plunger 40 which are inside the hollow barrel section.

Fig. 2 is an axial cross-sectional view which shows a configuration of the syringe barrel 20 according to one embodiment of the present invention. The syringe barrel 20 is a molded article by injection molding of a synthetic resin material like a polyolefin resin material, and comprises a tubular barrel section 21 for accommodating an internal-use solution in the interior thereof, an injection nozzle section 22 which is formed at one end of the barrel section 21 and to which a needle as an example is to be attached, and a flange section 23 which is formed at an opening section on the other end of the barrel section 21 to project radially outward. In this embodiment, as shown in Fig.1(B), when the internal-use solution is poured into the syringe barrel 20, a portion of the syringe barrel 20, which is filled by an internal-use solution 60, is called as an internal-use solution filled section 20F, and a portion which is not filled by the internal-use solution 60 is called as an internal-use solution unfilled section 20N.

In Fig, 2, the injection nozzle section 22 is formed as a Lure Lock type, wherein a screw tube part is connected to the outer side of a cylindrical nozzle part, the cylindrical nozzle part has a tapered surface, which is formed to be thinner toward the tip, on the outer circumference, and the screw tube part has a male screw on the inner wall surface. In this structure, by fitting an injection needle to the injection nozzle section 22 firmly, it is possible to certainly prevent the attached injection needle from becoming loose or being dislocated from the nozzle section, when it is used in a medical field or the like. Such a syringe barrel 20 may be used for mixed injection to a drip infusion portion, a two-liquid blending syringe between syringes or the like.

The syringe barrel 20 comprises an internal-use solution filled section 20F corresponding to the portion which is closer to the injection nozzle section 22 than a piston tip position 20P and an internal-use solution unfilled section 20N corresponding to the portion which is closer to the flange section 23 than the piston tip position 20P when a predetermined amount of the internal-use solution is put into the interior thereof.

As shown in Fig.1(A) and Fig. 2(A), a portion of the barrel section 21, which is close to the flange section 23, constitutes a large outer diameter barrel section 24, which has an outer diameter lager than the outer diameter of the intermediate body part 21M, and is formed between the flange section 23 and the intermediate body part 21M. It is considered that the relationship between the outer diameter D22 of the injection nozzle section 22, the outer diameter D21 of the intermediate body part 21M and the outer diameter D24 of the large outer diameter barrel section 24 may be D24=D22>D21, D24>D22>D21 or D22 > D24 > D21, however the relationship of D24 ≧ D22 > D21 is preferable.

As shown in Fig.1 (A) and Fig.2 (A),(B). the flange section 23 has two recess portions 23R, each has an approximately semielliptical notched shape and is formed at one of two positions on the outer periphery, respectively. When the flange section 23 is observed from the direction shown by the arow B in Fig.2(A), as shown in Fig.2(B), the inner wall surface 24N of the large outer diameter barrel section 24, the inner wall surface 21N of the intermediate body part 21M and the inner wall surface 22N of the nozzle part of the injection nozzle section 22 are viewed in this order concentrically from the outer surface.

Next, a manufacturing method for producing the syringe barrel 20 shown in Fig.2 will be explained referring to Fig. 3. As shown in Fig.3, a mold 300 is prepared for molding the syringe barrel 20. The mold 300 has a structure which can be divided appropriately for molding complicated shapes like the shapes of the injection nozzle section 22, the flange section 23 and the like. Although the mold 300 is shown as a mold for molding a single syringe barrel 20, it is possible to use a mold for molding plural syringe barrels 20 simultaneously. An injection molding machine, which is not shown in the drawings, has a mold closing mechanism to which a mold is installed and an injection equipment for filling a melted resin material.

The injection molding method comprises a mold closing step for closing an open mold by a mold closing mechanism, a melted resin filling step for filling a melted resin material into a closed mold by an injection molding machine, a molded article cooling step for cooling the resin material in the mold for a predetermined time after the melted resin filling step, and a molded article extracting step for extracting the molded article by opening the mold after the molded article cooling step is completed.

For example, a female die of the mold is installed to the injection equipment side (the fixed side) and a male die of the mold is installed to the mold closing mechanism side (the movable side). A melted resin material is injected into the cavity which is formed by engaging the male die of the mold with the female die of the mold. The injected melted resin material flows into the cavity through a gate from a spool in the mold. A molded article is released from the mold after cooling and solidifying the resin material which is filled in the mold.

As sown in Fig.3(A), a cavity 320, in which the syringe barrel 20 is to be molded, is formed by a mold 300. The cavity 320 comprises a cavity 322 corresponding to the injection nozzle section in which two cylindrical cavities are connected, a cylindrical cavity 321M corresponding to the intermediate body part, a cylindrical cavity 324 corresponding to the large outer diameter barrel section, and a cavity 323 corresponding to the flat plate shaped flange section. Also, the mold 300 has a shape for forming the recess portions 23R in the flange section 23.

In Fig.3(B), the mold for surrounding the outside of the cavity 323 corresponding to the flange section is omitted, and the cavity 323 corresponding to the flange section is a flat plate shaped cavity in which a portion corresponding to the inner wall surface 24N of the large outer diameter barrel section 24 is partitioned by the mold. Two convex portions 323C which project inwardly are formed on the inner wall surface of the mold 300 so that the two convex portions 323C penetrate the cavity 323 corresponding to the flange section at the two positions on the outer periphery.

As shown in Fig.3(C), the shape of the convex portions 323C which are formed at the two positions on the outer periphery of the cavity 323 corresponding to the flange section corresponds to the shape of the recess portions 23R. The gates 310 of the injection molding machine are installed at the positions where the recess portions 23R are formed, and the melted resin material is delivered into the cavity 320 in the mold 300 from the injection molding machine through the gates 310.

As shown in Fig.3(B), the cavity 323 corresponding to the flange section of the cavity 320 has a same shape as the flange section 23. When a melted resin material is injected from the outer periphery of the flat plate shaped cavity 323 corresponding to the flange section through the two gates 310, the flat plate shaped cavity 323 corresponding to the flange section will be filled with the melted resin material from the outer periphery toward the inner periphery, and the streams of the melted resin material will join together in the vicinity of the center line portion 323M which is sown by the dotted line. The filling balance is improved by installing the two gates 310 when the mold is filled with the melted resin material. For manufacturing a minimal capacity syringe barrel according to the present embodiment, it is possible to fill the mold with the melted resin material by installing one gate to four gates. If one gate is installed, it becomes difficult to keep the filling balance because the filling port is unbalanced. If three or more gates are installed, although the filling speed becomes higher, it requires a larger amount of melted resin material because the number of runners will increase. Therefore, it is preferable to install two gates in the present embodiment.

The melted resin material flows into the cavity 323 corresponding to the flange section first, and then flows into the cavity 324 corresponding to the large outer diameter barrel section. As shown in Fig.3(D), there is also a possibility that the streams of the melted resin material join together in the vicinity of the center line portion 324M of the cavity 324 corresponding to the large outer diameter barrel section which is shown by the dotted line. In Fig. 3(D), the outer and inner molds for the cavity 324 corresponding to the large outer diameter barrel section are omitted, the molds are installed at the inner circular portion and the outer portion which is not shown in the drawings.

After the cavity 320 is filled completely in the circumferential direction with the melted resin material, the streams of the melted resin material no longer join together in the circumferential direction, the melted resin material flows cylindrically toward the side of the cavity 322 corresponding to the injection nozzle section, and the melted resin filling step is completed by filling the cavity 322 corresponding to the injection nozzle section. Subsequently, a syringe barrel as a molded article shown in Fig.2 is obtained by conducting the molded article cooling step and the molded article extracting step.

In this embodiment, when the syringe barrel 20 is formed by injection molding of a synthetic resin material, as shown in Fig.3 (B), the streams of the melted resin material, which are injected from the two gates 310, join together in the vicinity of the center line portion 323M shown by the dotted line, by flowing around the circumference of the cylindrical male die of the mold while being cooled on the surface of the male die inside the inner wall 324N of the cavity 324 corresponding to the large outer diameter barrel section.
A linear pattern called as a weld line is formed at the merging part of the two streams of the resin material, wherein the weld line may cause occurrence of failure on its appearance and insufficient mechanical strength. The weld line is a phenomenon occurred by a slightly solidified resin material surface.

For creating a condition in which the surface of the merged melted resin material hardly solidifies, it is considered to delay the solidification by increasing the mold temperature, make the solidified layer on the surface thinner by increasing the injection speed, set the resin material temperature higher, set the resin material pressure higher and so on, however it is impossible to prevent occurrence of the weld line by these treatments.

In this manner, when the flange section 23 is formed, a weld line is formed in the vicinity of the center line 323M where the streams of the melted resin material join together. Next, there is also a possibility that a weld line is formed in the large outer diameter barrel section 24 when the melted resin material flows into the cavity 324 corresponding to the large outer diameter barrel section after filling the cavity 323 corresponding to the flange section and then the streams of the melted resin material join together in the vicinity of the center line 324M.

When the melted resin material fills the cavity 324 corresponding to the large outer diameter barrel section and extends through the entire circumference, the streams of the melted resin material no longer join together on the circumference at the time of flowing into the cavity 321M corresponding to the intermediate body part. Therefore, no weld line will be formed in the intermediate body part 21M.

Depending on the injection molding condition, there is also a possibility that the streams of the melted resin material join together on the circumference in a part of the cavity 321M corresponding to the intermediate body part which is close to the cavity 324 corresponding to the large outer diameter barrel section, and a weld line is potentially formed. However, no weld line is formed in a part of the cavity 321M corresponding to the intermediate body part which is close to the cavity 322 corresponding to the injection nozzle section because the streams of the melted resin material do not join together on the circumference.

Therefore, it is possible to obtain the internal-use solution filled section 20F where no weld line is formed by setting the part of the intermediate body part 21M, which is close to the large outer diameter barrel section 24, to the internal-use solution unfilled section 20N and the part of the intermediate body part 21M, which is close to the injection nozzle section 22, to the internal-use solution filled section 20F

According to this embodiment, because no weld line is formed in the internal-use solution filled section 20F, no gap is formed between the outer circumferential surface of the piston 50 and the inner wall surface of the syringe barrel 20, and thus no internal-use solution will leak out. Hereby, it is possible to obtain a syringe barrel which can fill and administer a predetermined amount of the internal-use solution accurately and is manufactured by injection molding of a synthetic resin material.

In the molded article extracting step, gate rests are formed when the gates are separated from the molded article. The gate rests are formed by leaving the solidified resin material at the portions of the molded article which correspond to the positions of the gates. When the portions of the molded article corresponding to the gates are not solidified, the positions of separating the gates become unstable and solidified resin material will remain at the portions of the separated molded article corresponding to the positions of the gates. Although it is considered that the gate rests can be made smaller by adjusting the gate shape, the injection time, the mold opening speed and the pressure applied to the vicinity of the gates and so on, it is practically unavoidable to form a certain size of gate rests.

In general, the gate rests are removed by a treatment like polishing because the projections by the gate rests may cause injury at the time of use by a medical worker and a failure in mounting a finger grip. However, it is not preferable to conduct such post processing for preventing foreign matter contamination and treating in a hygienical full-automatic production process.

In this embodiment, as shown in Fig.3(C). a convex portion 323C is formed in the cavity 323 corresponding to the flange section of the mold 300, and a gate 310 is positioned so that it corresponds to the convex portion 323C. When a medical syringe barrel of a capacity of 0.25mL is used as an example of the syringe barrel 20, the thickness of the flange section is set to 2mm, the length in the radial direction of the recess portion 23R is set to 4mm, and the depth of the recess portion 23R is set to 1mm. In this example, as shown in Fig.2(C), because the width and the height of a gate rest 25 fits inside the recess portion 23R even though the gate rest 25 is formed inside the recess portion 23R of the flange section 23 after the injection molding step, a medical worker will not be injured at the time of operation, and no inconvenience will occur at the time of attaching a finger grip. Therefore, it is not necessary to conduct a post processing treatment like polishing which is inconvenient for manufacturing a medical equipment.

According to the above-described embodiment, it is possible to provide a medical syringe as a molded article by injection molding of a synthetic resin material and a manufacturing method for producing the same, wherein it is possible to be completed without requiring a post processing treatment like polishing and therefore possible to fill and administer a predetermined amount of an internal-use solution accurately. A syringe barrel according to the present embodiment is suitable for a prefilled syringe, wherein a medical solution is filled in a syringe barrel and the syringe is assembled in advance. A syringe barrel according to this embodiment is also suitable for a disposable syringe which is assumed to be used a single time.

### Explanation of References

- 10: syringe
- 20: syringe barrel
- 20F: internal-use solution filled section
- 20N: internal-use solution unfilled section
- 20P: piston tip position
- 21: barrel section
- 21M: intermediate body part
- 21N: inner wall surface of the intermediate body part 21M
- 22: injection nozzle section
- 22N: inner wall surface of the nozzle part of the injection nozzle section 22
- 23: flange section
- 23R: recess portion
- 24: large outer diameter barrel section
- 24N: inner wall surface of the large outer diameter barrel section 24
- 25: gate rest
- 30: finger grip
- 300: mold
- 310: gate
- 320: cavity
- 321M: cavity corresponding to the intermediate body part
- 322: cavity corresponding to the injection nozzle section
- 323: cavity corresponding to the flange section
- 323C: convex portion
- 324: cavity corresponding to the large outer diameter barrel section
- 324N: inner wall of the cavity corresponding to the large outer diameter barrel section
- 40: syringe plunger
- 50: piston
- 60: internal-use solution

## Claims

1. A medical syringe barrel comprising a tubular barrel section for accommodating an internal-use solution in the interior thereof, an injection nozzle section formed at one end of the barrel section, and a flange section formed at an opening section on the other end of the barrel section to project radially outward, wherein
the barrel section includes an internal-use solution filled section which is set to be filled with the internal-use solution and an internal-use solution unfilled section which is set not to be filled with the internal-use solution when the internal-use solution is poured into the syringe barrel from the opening section;
the surface of the flange section has a recess portion; and
a weld line which is potentially formed by injection molding is not formed in the internal-use solution filled section.

2. A medical syringe barrel according to Claim 1, the height of a gate rest by injection molding is smaller than the depth of the recess portion in the flange section, and the gate rest fits in the interior of the recess portion.

3. A manufacturing method for producing a medical syringe barrel, which comprises a tubular barrel section for accommodating an internal-use solution in the interior thereof, an injection nozzle section formed at one end of the barrel section, and a flange section formed at an opening section of the other end of the barrel section to project radially outward, wherein
preparing a mold so that the surface of the flange section has a recess portion; and
conducting injection molding of a thermoplastic resin material by installing a gate at the position where the recess portion is to be formed.
